# EUROPEAN PATENT SPECIFICATION

(11) **EP 1 558 921 B1**
(45) Date of publication and mention of the grant of the patent: **20.10.2010**
(21) Application number: 03757747.5
(22) Date of filing: 29.10.2003
(51) Int. Cl.: G01N 27/00, G01N 33/00

(54) **METHOD OF PERFORMING CALIBRATION AND QUALITY CONTROL OF A SENSOR AND APPARATUS FOR PERFORMING SAID METHOD**
VERFAHREN ZUR DURCHFÜHRUNG DER KALIBRATION UND QUALITÄTSKONTROLLE EINES SENSORS UND VORRICHTUNG ZUR DURCHFÜHRUNG DES VERFAHRENS
PROCEDE DESTINE A L'ETALONNAGE ET AU CONTROLE DE LA QUALITE D'UN CAPTEUR ET DISPOSITIF DESTINE A LA MISE EN OEUVRE DE CE PROCEDE

(30) Priority: 30.10.2002 DK 200201647
(43) Date of publication of application: 03.08.2005
(73) Proprietor: Radiometer Medical ApS, 2700 Broenshoej (DK)
(72) Inventor: FRISCHAUF, Peter, Aage, DK-2605 Brøndby (DK); LARSEN, Eiler, DK-2605 Brøndby (DK)
(86) International application number: PCT/DK2003/000728
(87) International publication number: WO 2004/040284

(56) References cited:
- WO-A-02/066973
- WO-A-03/019165
- WO-A2-03/019165
- US-A- 4 163 734
- US-A- 4 469 792
- US-A- 4 786 394
- US-A- 5 424 212
- US-A- 6 037 178

## Description

The present invention relates to a method of performing calibration and quality control of a sensor for determining a parameter in a test fluid as well as an apparatus for performing said method.

Sensors for measuring a parameter in a test fluid are widely used in various fields of industries. Examples of industries are the food industry, the environmental industry and the medical and the clinical industry, in particular the clinical laboratory industry.

In order to assure that sensor measurements are accurate it is required to regularly calibrate the sensors, i.e. to determine experimentally the correspondence between sensor responses and predetermined parameter values of the reference materials, usually by measurements on one or more reference materials.

In many cases it is also required to regularly control the quality of the sensor performance, i.e. to verify experimentally that the sensor measurements are accurate and/or precise, usually by comparison of a measured parameter value of a reference material with an acceptance range of the same reference material.

In the food industry such routines of calibration and quality control are performed for instance on sensors for assessing milk quality. In the medical and clinical industry such calibration and quality control routines are performed for instance on sensors for determining parameters in physiological fluids.

US 6,171,865 B1 discloses a reference sensor system comprising a laminar flow channel in which during analysis sample, indicator and reference streams flow. In the method described separate calibration streams are used to calibrate the system. The reference stream may serve as both a control stream and an internal standard stream. That is the measurement on the reference stream is used first in the quality control and if it falls within the acceptance range the same measurement may be used as internal standard measurement.

Similarly, DE 41 04 302 discloses a method of performing quality control of and optionally calibrating a sensor for determining a parameter in a physiological fluid. According to the method one and the same volume of reference solution may be used to control and, if needed, to calibrate a measuring cell. The advantage is that the same reference solution may be used both in measuring cells for determining the hematocrit value and in cells for determining physiological electrolytes. In the method, however, the same volume of reference solution is used for both calibration and quality control of the sensor.

US 5424212 discloses a sensor calibration followed by a quality control carried out by means of a control solution which is usually different from the calibration solution.

In none of these documents are there any suggestions or hints towards using a reference material for quality control of a sensor, which at another stage is used for calibration of the sensor in a way, which retains an independent assessment of sensor performance.

The general requirement for reference materials for performing calibration and quality control routines is, that the reference material used in quality control of a sensor must be different from the reference material(s) used for the calibration upon which the quality control is based in order to ensure that the quality control procedure provides an independent assessment of sensor performance.

In the clinical and medical industry legislation requires that sensors are exposed to quality control procedures in addition to regular calibration procedures. The quality control procedures must be performed often enough and cover a broad enough parameter range to be able to prove, at any time, that the sensor used is able to provide reliable data in its entire measuring range. This means that sensors must be checked by means of a system, which is independent of the normal calibration system of the sensor.

It is accordingly recommended that in successive quality control cycles the quality of a sensor is controlled using a number of reference materials representing different parameter levels (often low, middle and high).

The same reference material may represent several different parameters at a time. This way calibration and/or quality control may be performed on several sensors simultaneously using only one reference material. One level of one parameter may be combined with any level of other parameters. An example of such a reference material is found in US 5,910,445 disclosing quality control liquids for use in the quality control procedure of electrochemical measuring apparatuses for measurement on physiological liquids.

Further, US 4,701,417 discloses calibration and control sera for use in lipid diagnosis. Solutions for calibrating and/or quality controlling sensors in blood analysers are disclosed in for example US 5,422,278 and US 5,910,445. In all cases the reference materials for calibration and the materials for quality control are described as two separate groups of reference materials.

In automatic apparatuses for blood analysis it is standard procedure to use at least five different reference materials in the calibration and quality control of the respective sensors. An example is the ABL™725 blood analyser manufactured by Radiometer Medical A/S. In this apparatus several different blood parameters are determined. It contains two calibration solution containers which are integrated in the apparatus and which need to be renewed approximately once a month.

In the ABL™725 blood analyser the quality control may be performed manually by a specially trained operator at specific hours by aspirating reference solution from an ampoule. After shaking and breaking open the ampoule, it is provided with an adapter for attaching it to the inlet upon which an automated quality control procedure is initiated. When the operator has obtained a measuring result, he or she must determine whether the result falls within a certain acceptance range, which is given on an insert of the ampoule. It is recommended that reference materials of at least three different parameter levels are used.

Alternatively, the ABL™725 blood analyser may be provided with a module ' (AutoCheck™) for automatic aspiration of control fluid from an ampoule for quality control of the apparatus. With this module operator handling is reduced to removing a holder from the apparatus, loading the holder with ampoules and reinserting it. This must be done approximately once a week. The operator still needs to pay attention to the number of ampoules of each level that he or she loads into the holder.

Thus, in the known methods of performing calibration and quality control of a sensor for determining a parameter in a test fluid, a substantive number of different reference materials are required. Also, the known methods require that a specially trained operator perform the quality control routine or at least once a week load a holder with ampoules for automatic aspiration of quality control fluid.

One object of the present invention is to provide a method of performing calibration and quality control of a sensor for determining a parameter in a test fluid in which method the number of reference materials may be reduced yet retaining the same precision and accuracy in the calibration and quality control of the sensor and yet fulfilling the requirement that the reference material used in the quality control is different from the reference material(s) used in the calibration upon which the quality control is based.

Another object of the present invention is to provide an apparatus for determining a parameter in a test fluid in which the apparatus employs the method according to the present invention for performing calibration and quality control of a sensor contained in the apparatus.

A further object of the present invention to provide a method and an apparatus which allow automated handling of the quality control procedure of a sensor. Thus the required maintenance and the need for specially trained operators for operating such apparatuses is minimised. Moreover the risk of human errors is minimised as well.

According to the invention the object is achieved by providing a method of performing calibration and quality control of a sensor for determining a parameter in a test fluid which method is defined in claim 1

The method according to the present invention has the advantage that the number of reference materials can be reduced by using the same reference materials both for calibration and for quality control purposes without reducing the quality of the calibration and the quality control and yet retaining the independence between the calibration system and the quality control system. The only requirement is that one and the same reference material is not used both in the quality control step and in the calibration step upon which the quality control is based.

A reduction in the number of reference materials makes it possible to reduce the storage space for reference material. If this space is inside an apparatus provided with the sensor, the size of the apparatus may be reduced.

The fully automated handling of the quality control procedure of a sensor has the advantage that the requirement of specially trained operators for operating such apparatuses as well as the risk of human errors are minimised.

The test fluid to be analysed may be any fluid in which it is desired to determine a parameter. Examples are fluids in food processing, such as beer, milk and meat processing fluids, and physiological fluids, such as whole blood, blood plasma, serum, cerebrospinal fluids, spit and urine.

The parameter towards which the sensor may be specific may be any parameter of interest, for example:
viscosity, density, pressure, conductivity, and surface tension.

Of particular interest are parameters of physiological fluids such as pH, concentrations of electrolytes, such as Li⁺, Na⁺, K⁺, Ca²⁺, Mg²⁺, Cl⁻, HCO₃⁻ and NH₃ (NH₄⁺), concentrations of dissolved gases, notably oxygen and carbon dioxide (conventionally reported in the form of partial pressures, e.g. *p*O₂, *p*CO₂), hematocrit (Hct), concentration of haemoglobin and haemoglobin derivatives, such as oxyhaemoglobin, deoxyhaemoglobin, methaemoglobin, carboxyhaemo-globin, sulfhaemoglobin and fetal haemoglobin, concentrations of metabolic factors, such as glucose, creatinine, creatine, urea (BUN), uric acid, lactic acid, pyruvic acid, ascorbic acid, phosphate, protein, bilirubin, cholesterol, triglycerides, phenylalanine and tyrosine, concentrations of enzymes, such as lactic acid dehydrogenase (LDH), lipase, amylase, choline esterase, alkaline phosphatase, acid phosphatase, alanine amino transferase (ALAT), aspartate amino transferase (ASAT) and creatinine kinase (CK), and concentrations of ligands, such as antibodies and nucleotide fragments.

As used here, the term "sensor" denotes any kind of device of which some part, in the present context called the sensing part, is capable
either of
selectively interacting with the chemical species of interest, thereby producing a welldefined and measurable response which is a function of the desired characteristic of that chemical species, the desired characteristic thus being derivable therefrom,
or of
responding to a bulk property of a fluid, the response not being selective with respect to any specific chemical species, but being a function of the total concentration of one or more chemical species in the liquid, the desired characteristic thus being derivable therefrom.

Relevant types of sensors are those adapted to determine any of the previously mentioned parameters, for example:
potentiometric sensors for use in aqueous media, such as ion-selective electrodes for specific measurement of the concentration of selected ionic chemical species, the response being in the form of an electric potential, amperometric sensors, such as sensors for the determination of oxygen partial pressure, whose response is in the form of an electric current, optical sensors, such as sensors producing a colour response to a particular chemical species, the response being measured spectrometrically using reflectance or transmittance, and the colour response being a colour intensity or the decay of colour intensity, piezoelectric sensors, thermometric sensors, pressure-change sensors, acoustic sensors, enzyme-based sensors employing an enzymatic reaction and generating a response on the basis of any relevant physical principle, for example any of those principles employed in the sensor types listed above; examples are enzyme-based thermistors and enzyme-based amperometric sensors for use in the measurement of concentrations of metabolic products, e.g. glucose, urea, creatinine or lactate, and affinity sensors comprising one moiety of an affinity pair, e.g. an antigen/antibody pair or two complementary nucleotide fragments, the other moiety being the chemical species of interest.

The sensor may be of any design. Accordingly both miniaturized, planar sensors, and conventional sensors are suitably calibrated and quality controlled by the method according to the present invention.

Sensors generally perform a conversion function to convert the energy form associated with the change occurring at the surface of the sensing part to electrical energy or electromagnetic radiant energy, the sensor response thereby being registerable in the form of an electrical or optical signal.

The measured sensor response may be a one-point measurement, an average of a slightly varying signal, a transient or an estimate based on a transient. It may be preferable to obtain several responses and use an average of these.

A "calibration" of the sensor is to be understood as an experimental determination of the correspondence between the sensor responses and predetermined parameter values of a reference material. Usually, said correspondence is found by obtaining sensor responses to one or more reference materials having predetermined parameter values and determining the correspondence between those.

The correspondence is most often expressed as a regression function calculated from the predetermined parameter values and the corresponding sensor responses. Alternatively, if the measuring results are shown directly on a graduated scale, the graduated scale may be moved to show the predetermined parameter value of the reference material.

The correspondence determined in the above calibration is then used when a parameter in a test fluid is to be determined. First a sensor response to the test fluid is obtained. Then the sensor response is converted into a measured parameter value by using the correspondence determined.

The conversion may be effected by programmed control means comprising an algorithm to provide a measured parameter value. The algorithm is adjusted in each calibration step.

Any number of reference materials may be used in the calibration step. The number of reference materials which are required to obtain a reliable calibration of a sensor depend on the nature of the sensor and of the demands for accuracy and/or precision. It is thus preferred to use reference materials representing one to five different parameter levels in the calibration step. Two or three different levels are more preferred in many instances, since this for most sensors provides sufficiently reliable results and at the same time limits the number of different reference materials. For some sensors , e.g. many biosensors, it is however required to use four or five reference materials to obtain sufficiently reliable results.

It may be sufficient to initially calibrate the sensor once using more than one reference material. Any subsequent calibrations may then be performed using only one reference material and is simply used to correct the previously determined correspondence between sensor responses and predetermined parameter values.

However many sensors need to be calibrated regularly and often using reference materials representing at least two parameter levels. A calibration using reference materials representing more than two parameter levels may in some cases provide a more reliable calibration. For instance, oximetry modules are often calibrated in one point, many ion selective sensors in two points, and many enzyme sensors in three points.

One of the reference materials used in the calibration of the sensor may be a blank. That is, it may represent a parameter level of "zero".

A "quality control" of the sensor is to be understood as the experimental verification that the sensor measurements are accurate and/or precise. Usually such verification is performed by determining whether a measured parameter value of a reference material is within an acceptance range thereof. The measured parameter value of the reference material is obtained by converting the sensor response into the measured parameter value using a calibration correspondence as described above. It is then determined whether the measured parameter value is within the acceptance range of the reference material.

The acceptance range is generally centered around a predetermined parameter value. The limits of the range depend e.g. on sensor variation, on the variation when determining the predetermined parameter value of the reference materials for both the quality control and the calibration and/or demands for accuracy and precision.

A "calibration and quality control cycle" is to be understood as one calibration and one quality control where the correspondence determined in the calibration is used in the cycle for converting the sensor response to the reference material of the quality control into a measured parameter value.

In the method all reference materials may be used in each calibration and quality control cycle. If a total of more than three reference materials are used in the method all may be used in each calibration and quality control cycle, or a selection among the total number of reference materials may be used in each cycle. The only demand is that the total number of reference materials at least equals the number of quality control levels desired.

There is no demand as to any time interval between the calibration and the quality control, nor to the time interval between two cycles. If it is desired to control performance of the sensor and the apparatus as well as the quality of the reference materials, the quality control should preferably be performed fairly shortly after the calibration upon which the quality control is based. If it is desired to control the drift of a sensor within a certain period of time the calibration upon which the quality control is based should preferably be performed at the beginning of the period and the quality control at the end of the period.

Between one cycle of calibration and quality control and another cycle of calibration and quality control supplementary calibrations and/or quality controls may be performed on the same or different reference materials.

In this context the "another cycle" denotes a cycle, which is carried out any number of cycles before or after the cycle in question, that is a previous or subsequent cycle.

A "reference material" is a material representing an exact parameter value. It is preferred that the reference material retains a constant value for an extended period of time. Reference materials for use in the same cycle must be prepared independently of each other in order to provide an independent assessment of sensor performance.

Such reference material preferably has a "predetermined parameter value". This may be the experimental determination of the parameter value itself, or it may be the same adjusted to take into account various systematic errors in the measuring method. Examples of such errors affecting systematically the measured result are the nature of the test fluid and the apparatus and the sensor with which the test fluid is analysed as well as residues near the sensor surface and measurements performed before equilibrium is obtained at the sensor surface.

Since the procedure of performing the calibration may deviate from that of the quality control, one reference material may have one predetermined parameter value for use in the quality control and another predetermined parameter value for use in the calibration, the two values taking into account different systematic errors.

A reference material may be referred to as representing or having a parameter "level". This level is to be understood as an approximate parameter value of a given reference material, the exact parameter value lying in the neighbourhood of the level.

The reference materials may be in fluid or solid form and contain or mimic any of the parameters mentioned earlier. If in fluid form, the reference materials are preferably provided in sealed containers.

In apparatuses designed for analysing fluids, it is preferable that the reference materials are fluids, for example a liquid, a gas or a combination thereof.

If the reference material is a fluid, the fluid may also contain constituents having a rinsing action. Thus a separate rinsing or cleaning solution may be omitted or used more rarely.

For some optical sensors the reference materials may be a solid brick or filter comprising a colour inducing material.

In a full calibration and quality control routine, the cycle as defined in claim 1 is repeated until all available reference materials have been used at least once for the quality control of the sensor performance. Thus the performance of the sensor is controlled at all the different parameter levels in one routine providing a more reliable quality control of the sensor.

Preferably this routine is systematically repeated, making the method even more suitable for being automatically performed.

A reference material may represent more than one parameter. This is an advantage if it is desired to determine several different parameters in one sample of test fluid and thus to calibrate or quality control more than one sensors simultaneously. If the reference material represents different parameters at various relevant levels it is possible to calibrate or quality control all sensors at one level in one operation. Many automatic analysers perform simultaneous multi analyte determination of a test fluid. One example is a blood analyser, such as the above-mentioned ABL™725.

The total range of the parameter levels of the reference materials should in the present methods essentially cover the specified measuring range of the sensor. This is in order to ascertain that the measurements are reliable in that range.

Also, the distribution of the respective parameter levels of the various reference materials within the total range may influence the reliability of the quality control. If the correspondence determined in the calibration is used for conversion of a sensor response into a measured parameter value that lies outside the calibration range, the uncertainty of the determination will be greater the farther away from the calibration the measured or predetermined parameter value is.

If in a cycle the parameter levels of the reference materials used for performing the calibration upon which the quality control is based define a calibration range, and the parameter levels of all the reference materials used in the cycle define a total range, it is preferred that the calibration range is at least one fourth of said total range. It is particularly preferred that the calibration range is at least one third of the total range.

The methods according to the present invention are particularly suitable for performing calibration and quality control of a sensor for determining a blood parameter, since it fulfils the legal requirements to calibration and quality control in the clinical field, is reliable and requires only limited maintenance from the operator and not at specific hours of the day. It will often be sufficient to renew the containers of reference fluid once or twice a month.

Further the containers may be provided in a cartridge and thus can be renewed without the operator having any prior knowledge as to the choice of containers or to the correct connection of the containers to the apparatus.

Thus, according to a preferred embodiment of the present invention the parameter to be determined is a blood parameter, such as pH, *p*CO₂ and *p*O₂, electrolytes such as Li⁺, Na⁺, K⁺, Ca⁺⁺, Mg⁺⁺, Cl⁻, HCO₃⁻ and NH₄⁺, haemoglobin, haemoglobin derivatives, Hct, or metabolic factors, such as bilirubin, glucose, lactate or creatinine.

According to another aspect of the present invention, an apparatus is provided for determining a parameter in a test fluid comprising
a sensor sensitive to the parameter in the test fluid and providing a sensor response,
reference materials representing at least three different parameter levels of the parameter,
a programmable device for controlling the functioning of the apparatus
the programmable device controlling the execution of the method of performing a calibration and quality control of the sensor according to the methods of the present invention:

The apparatus according to the present invention has the advantage that the number of reference materials may be reduced yet retaining the same precision and accuracy in the calibration and quality control and yet fulfilling the requirement that the reference material used in the quality control based upon a calibration is different from the reference material(s) used in the same calibration.

A reduction in the number of reference materials makes it possible to reduce the storage space for reference material. Inside the apparatus the storage requirement may also be reduced thus reducing the size of the apparatus.

Further, the methods according to the present invention provide the apparatus with a fully automated quality control routine, thus minimising the maintenance requirements and need for specially trained operators for operating such apparatuses. Also, the risk of human error is minimised.

The terms "parameter", "test fluid", "sensor", and "reference material" are to be understood as having the same meanings as explained above.

The sensor "response" is to be understood as the energy formed in association with the change occurring at the sensing surface or the energy converted into electrical energy or electromagnetic radiant energy which is registerable by conventional converters.

The "programmable device" carrying out the functions and operations of the apparatus may include, for example, a microprocessor or parallel processor, a memory bus, random access memory (RAM), and read only memory (ROM). The processor may be a general purpose digital processor which controls the operation of the programmable device. Using instructions retrieved from memory, the processor controls the reception and manipulation of input data and the output and display data. However, those skilled in the art will appreciate that the methods and system of the present invention may be advantageously implemented in a variety of hardware configurations.

The function and operation of the apparatus which the device may be programmed to control includes, for example, control of the means for exposing the sensor to test fluid and reference materials and recording and processing of sensor responses. The processing of sensor responses may for example include determining a calibration correspondence on the basis of recorded sensor responses corresponding to calibration material(s), comparing a recorded sensor response corresponding to a quality control material with a calibration correspondence, and obtaining measured parameter values from recorded sensor responses on the basis of a determined calibration correspondence. Thus the programmable device preferably comprises means for determining the measured parameter value.

To process a sensor response of the sensor into a measured parameter value the apparatus preferably includes an operational amplifier for amplifying and converting the sensor response as well as an appropriate algorithm and software for executing the processing of the signal. The choice of an amplifier for amplifying the sensor response naturally depends on the nature of the sensor. The algorithm for converting the amplified sensor response into a measured parameter value may comprise the correspondence determined in the calibration and appropriate adjustments taking into account diverse systematic errors in the measuring method. Examples are given above.

The apparatus according to the present invention will in general also comprise means for exposing the sensor to the test fluid, means for exposing the sensor to the reference material, means for obtaining a response and means for reporting a measured parameter value.

The "means for exposing the sensor to the test fluid" may for instance comprise inlet means for introducing the test fluid into the apparatus. The inlet means may be designed to receive the outlet opening of a sample container such as a syringe or a capillary or a specially designed sample container.

The means for exposing the sensor to the test fluid may further comprise an inlet probe for aspirating the sample of test fluid into the apparatus. Alternatively, the inlet may lead directly to a measuring chamber. The inlet or the inlet probe may be in fluid tight communication with a conduit. The conduit may comprise measuring chambers. It is preferably a tubing or a channel providing a fluid-tight path for transport of fluid through the conduit. The fluid may be transported through the conduit by means of a pump.

The sensor is exposed to the test fluid, either directly in the conduit or in a measuring chamber. The exposure of the sensor to the test fluid may be a direct physical contact between the sensing surface and the test fluid or it may be an indirect contact, the wall of the conduit or the measuring chamber separating the sensing surface from the test fluid. If optical sensing methods are employed, this part of the wall should be transparent to the type of radiation used.

The reference materials may be of the nature described above and may for example contain any of the parameters mentioned above. A total of two or three reference materials are preferred, but one or more further reference materials for calibrating the sensor may be included.

The "means for exposing the sensor to the reference material" may be the same as the "means for exposing the sensor to the test fluid". It should however also include a mechanical device, which is capable of moving reference material from a storage location to a location providing operational communication between the sensor surface and the reference material. If the reference materials are fluid the device is preferably a pump, if they are solid the device is preferably a robot arm or the like.

The "means for reporting a measured parameter value" may for example be a scale, a display, a printer, or a digital voice reporting the measured parameter value obtained from the processing means.

The apparatus may comprise more than one sensor. Preferably each sensor is specific towards one of the above-mentioned parameters.

The sensors may be miniaturized planar sensors. These may be located in a cassette for simultaneous renewal of all sensors.

The parts of such an apparatus being in contact with test fluid and/or reference fluid are often referred to as belonging to the "wet section" of the apparatus. This wet section may be an integral part of the apparatus or it may be provided in the form of a separate unit such as a cassette. The cassette may comprise several measuring chambers and/or sensors.

Preferably all the reference materials are located in a cartridge. Thus instead of changing one reference material at a time, all two or more reference materials may be renewed in one operation. This provides a more user-friendly renewal of reference materials, particularly if the reference materials are fluids provided in containers. The cartridge may also contain a waste container into which the used reference materials and the used samples of test fluid are discarded.

When a parameter level of a test fluid is to be determined a sample of the test fluid is introduced into an apparatus as described above and the analysis of the sample is being performed automatically. The reported measured parameter value is obtained from the apparatus.

The apparatus according to the present invention is preferably a blood analyser.

The methods and apparatus of the present invention may be used in a number of different settings including, for example, an industrial laboratory, a clinic or hospital, a research center or a university. Besides the normal use of the apparatus in such settings, the methods according to the present invention may also be practised during the manufacture, testing, sale and installation of the apparatus.

The invention will now be explained in detail by means of examples of embodiments and with reference to the drawing in which
Fig. 1 is a schematic drawing of the modified wet section of an automatic blood analyser of the present invention
Fig. 2 is a chart representing the first calibration curve and corresponding quality control as well as the second calibration curve for a pH sensor.

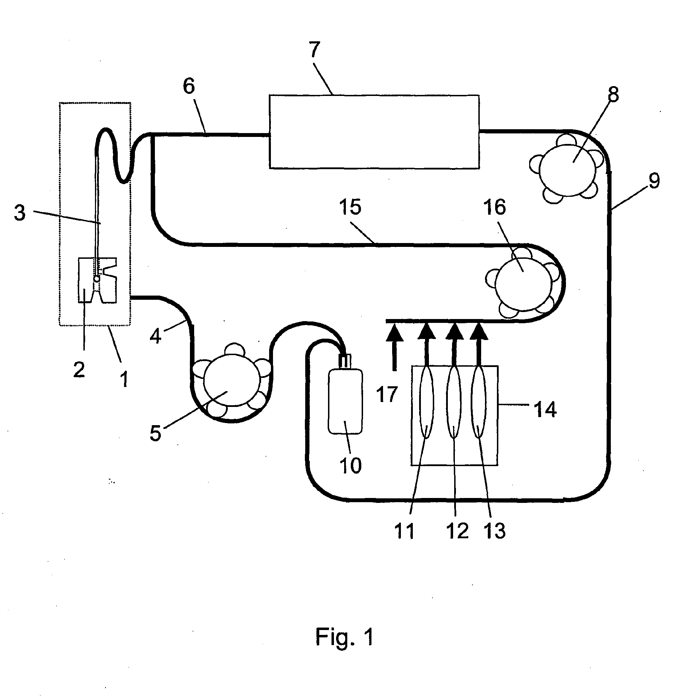

According to a preferred embodiment of the present invention the methods of performing calibration and quality control of a sensor are applied to a blood analyser having a wet section as shown in Fig. 1.

In Fig. 1 an inlet module 1 provides test fluid access to the apparatus. The inlet module 1 comprises an inlet gasket 2 provided with a cone for receiving the Luer of a syringe and a cone for receiving a capillary. A channel is leading from the cones through the gasket 2 to an inlet probe 3. If a syringe containing test fluid is connected to the inlet gasket 2, the inlet probe 3 may be displaced into the syringe and a sample of test fluid aspirated into the apparatus. The inlet gasket 2 is further provided with a tubing 4 and pump 5 for collecting any sample spills into a waste container 10.

The sample is transported through the tubing 6 to a measuring section 7 by means of pumps positioned after the measuring section, the pumps being represented by the pump 8. The measuring section 7 is thermostatically controlled at 37 °C. The measuring section 7 comprises sensors selective towards pH, CO₂, O₂, Cl, Ca, Na, K, glucose, lactate sensors, a reference sensor, and an oxymetry module.

After analysis the sample is transported through the tubing 9 by means of the pump 8 to the waste container 10.

The wet section further comprises a liquid cartridge 14 containing reference fluid containers 11, 12 or 13, tubing 15, a pump 16, and an air inlet 17.

When a calibration or quality control measurement is performed, a sample of reference fluid is transported from one of the respective reference fluid containers 11, 12 or 13, contained in the liquid cartridge 14 through the tubing 15 to the measuring section 7 by means of the pumps 16 and 8. After analysis the sample is transported to the waste container 10 as described above.

After each measurement a rinsing procedure is performed. The measuring section 7 is flushed with one of the reference fluids 11, 12 or 13 further comprising components with rinsing action. The air inlet 17 is used for introducing air bubbles into the stream of calibration solution when a rinsing procedure is performed. This provides a better cleaning action.

In the following non-limiting example, reference fluids are prepared as described in US 5,910,445. The compositions are as follows:

**Table 1: Compositions of reference fluids.**

| chemical species | Ref. 1 | Ref. 2 | Ref. 3 | Unit |
|---|---|---|---|---|
| MOPS | 51.9642 | 27.9425 | 37.5593 | mmol/kg H₂O |
| NaOH | 32.8466 | 9.0839 | 30.1676 | mmol/kg H₂O |
| NaCl | 104.5205 | 61.7754 | 142.1083 | mmol/kg H₂O |
| KCI | 4.2818 | 7.5475 | 2.1564 | mmol/kg H₂O |
| CaCl₂ | 0.9587 | 1.5985 | 0.3875 | mmol/kg H₂O |
| NaHCO₃ | 11.7346 | 12.3584 | 9.4234 | mmol/kg H₂O |
| glucose | 0.0 | 15.4784 | 7.2232 | mmol/kg H₂O |
| Na-lactate | 0.0 | 9.6192 | 4.8096 | mmol/kg H₂O |
| Na-sulforhodamin | 0.0 | 1.6705 | 2.5059 | mmol/kg H₂O |

The above compositions provide reference fluids with predetermined parameter values for calibration purposes as given below in table 2:

**Table 2: Predetermined parameter values (PPV) of reference fluids for calibration purposes.**

| Parameter | Ref. 1 | Ref. 2 | Ref. 3 | Unit |
|---|---|---|---|---|
| pH | 7.200 | 6.800 | 7.600 | |
| *p*CO₂ | 30.0 | 70.0 | 10.0 | mmHg |
| *p*O₂ | 170.0 | 90.0 | 50.0 | mmHg |
| cK⁺ | 4.0 | 7.0 | 2.0 | mmol/L |
| cNa⁺ | 140.0 | 90.0 | 180.0 | mmol/lL |
| cCa⁺⁺ | 0.8 | 1.65 | 0.40 | mmol/L |
| *c*Cl⁻ | 100.0 | 65.0 | 130.0 | mmol/L |
| *c*Glu | 0 | 15 | 7 | mmol/L |
| *c*Lac | 0 | 8 | 4 | mmol/L |
| *t*Hb | 0 | 9 | 12 | mmol/L |

The acceptance ranges of these reference fluids for quality control purposes are as follows:

**Table 3: Acceptance ranges (PPV) of reference fluids for quality control purposes.**

| Parameter | Ref. 1 | Ref. 2 | Ref. 3 | Unit |
|---|---|---|---|---|
| pH | 7.163 - 7.203 | 6.785 - 6.825 | 7.560 - 7.600 | |
| *p*CO₂ | 26.4 - 32.4 | 61.8 - 71.8 | 8.8 - 12.8 | mmHg |
| *p*O₂ | 158 - 178 | 82-102 | 55 - 75 | mmHg |
| *c*K⁺ | 3.3 - 3.9 | 6.0 - 6.6 | 1.7 - 2.3 | mmol/L |
| *c*Na⁺ | 125 - 134 | 75 - 85 | 163 - 168 | mmol/lL |
| *c*Ca⁺⁺ | 0.69 - 0.89 | 1.48 - 1.68 | 0.33 - 0.53 | mmol/L |
| *c*Cl⁻ | 90 - 102 | 61.0 - 73.0 | 120 - 132 | mmol/L |
| *c*Glu | 0 - 1 | 13.1 - 15.1 | 5.4 - 7.4 | mmol/L |
| *c*Lac | 0 - 1 | 6.4 - 7.4 | 2.7 - 3.7 | mmol/L |
| *t*Hb | 0 - 0.6 | 8.4 - 9.6 | 11.3 - 12.7 | mmol/L |

In an embodiment which is not according to the present invention, a first cycle of calibration and quality control is initialised by running an analysis of each of the three reference fluids Ref. 1, Ref. 2 and Ref 3.

The wet section including measuring chambers is flushed with reference fluid Ref. 1 in an amount corresponding to approximately twice the volume of the wet section. The wet section is filled again upon which sensor measurements are recorded every second for 30 seconds. This is a calibration measurement (Cal).

The above is repeated with Ref. 2.

Then the wet section is filled once with a sample of reference fluid Ref. 3 and sensor measurements are recorded every second for 30 seconds. The wet section is then flushed with reference fluid Ref. 3 in an amount corresponding to approximately the volume of the wet section. The wet section is filled again with Ref. 3, and sensor measurements are recorded every second for another 30 seconds. The first of the measurements is a quality control measurement (QC) and the second measurement is a calibration measurement (Cal).

After approximately eight hours the above cycle is repeated except that the double measurement is performed on Ref 2. After another eight hours the above cycle is repeated except that the double measurement is performed on Ref 1

In the following, the example is explained only with regard to the parameter pH. However the results of the measurements of any of the other parameters may be processed similarly. The obtained measured parameter values of pH are:

**Table 4: Measured parameter values of pH in cycle 1, 2 and 3.**

| | Ref. 1 | Ref. 2 | Ref. 3 | Ref. 1 | Ref. 2 | Ref. 3 |
|---|---|---|---|---|---|---|
| | QC measurements | | | calibration measurements | | |
| Cycle 1 | - | - | 7.575 | 7.195 | 6.804 | 7.602 |
| Cycle 2 | - | 6.805 | - | 7.200 | 6.800 | 7.601 |
| Cycle 3 | 7.189 | - | - | 7.205 | 6.798 | 7.605 |

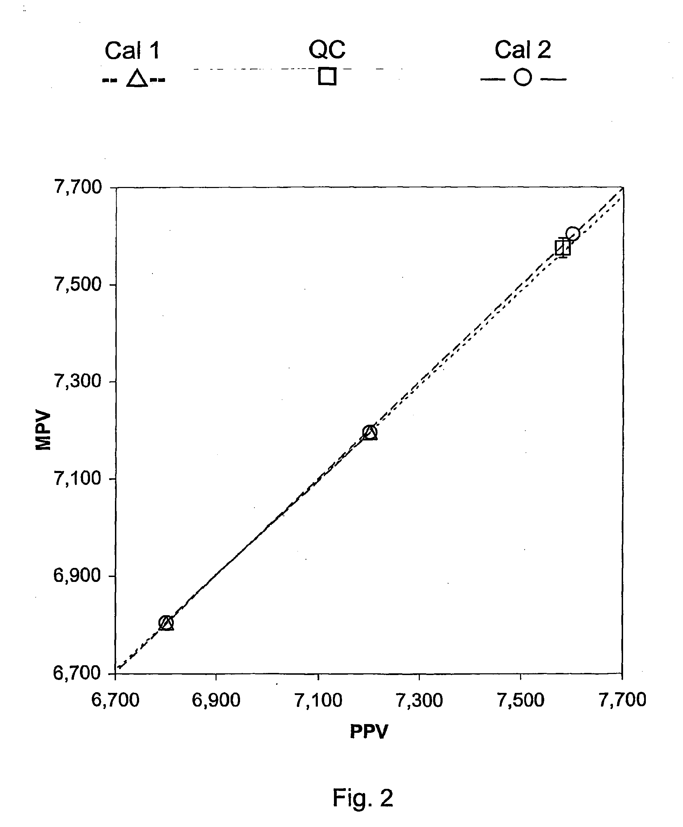

In cycle 1 a first calibration is obtained by estimating the correspondence between the predetermined parameter values (PPV) and the measured parameter values (MPV) of Ref. 1 and Ref. 2. The measured QC parameter level (MPV) of Ref. 3, which is obtained using the first calibration, is then compared to the assigned parameter level (PPV) with acceptance limits. This is depicted in Fig. 2.

As shown in Fig. 2 the QC parameter level of pH is within the acceptance limits and the quality control is accepted.

Subsequently, a second calibration is obtained based on all three calibration measurements, which second calibration may be used in determining measured parameter values of a test fluid. This second calibration curve is also depicted in Fig. 2.

The results of the two other cycles may be processed similarly. All quality controls fall within the acceptance limits as can be deduced from table 4.

According to the present invention, if three reference materials are used, the full routine of calibration and quality control comprises at least three cycles. The reference materials may be combined in a number of ways. A few combinations are exemplified below. The cycles A, B and C may be performed in any order.

**Table 5. In all cycles the quality control is based on a two-point calibration.**

| Cycle number | Ref. 1 | Ref. 2 | Ref. 3 |
|---|---|---|---|
| A | QC | cal | cal |
| B | cal | cal | QC |
| C | cal | QC | cal |

If more than three reference materials are used in the method, a further calibration point may be introduced and/or a second quality control may be performed. Optionally, some cycles of a full routine of calibration and quality control may include the further reference material(s) whereas they are left out in other cycles.

If four reference materials are used, the full routine of calibration and quality control comprises at least four cycles. The reference materials may be combined in a number of ways. A few combinations are exemplified below. The cycles A, B, C and D may be performed in any order.

**Table 9. In all cycles a three-point calibration is performed.**

| Cycle number | Ref. 1 | Ref. 2 | Ref. 3 | Ref. 4 |
|---|---|---|---|---|
| A | cal | cal | cal | QC |
| B | cal | cal | QC | cal |
| C | cal | QC | cal | cal |
| D | QC | cal | cal | cal |

**Table 10. Four reference materials are used, but in every cycle one reference material is a "dummy".**

| Cycle number | Ref. 1 | Ref. 2 | Ref. 3 | Ref. 4 |
|---|---|---|---|---|
| A | - | QC | cal | cal |
| B | cal | - | QC | cal |
| C | cal | al | - | QC |
| D | QC | cal | cal | - |

**Table 11. Quality control is performed at two levels in every cycle.**

| Cycle number | Ref. 1 | Ref. 2 | Ref. 3 | Ref. 4 |
|---|---|---|---|---|
| A | QC | QC | cal | cal |
| B | cal | QC | QC | cal |
| C | cal | cal | QC | QC |
| D | QC | cal | cal | QC |

**Table 12. In some areas of the total range it is preferred that a three-point calibration is performed, in others a two-point calibration is sufficient.**

| Cycle number | Ref. 1 | Ref. 2 | Ref. 3 | Ref. 4 |
|---|---|---|---|---|
| A | cal | QC | cal | cal |
| B | cal | - | QC | cal |
| C | cal | al | - | QC |
| D | QC | cal | cal | - |

## Claims

1. A method of performing calibration and quality control of a sensor for determining a parameter in a test fluid in which method a calibration and quality control cycle is repeated, the cycle comprising the steps of
performing a calibration of the sensor using reference materials representing different parameter levels of the parameter,
performing a quality control of the sensor as calibrated using another reference material representing another parameter level of the parameter than in the calibration step,
in which method
the reference material used in the quality control step of one cycle is used in the calibration step of each of the other cycles, the cycles being repeated until each of the reference materials has been used once for quality control of the sensor.

2. The method according to claim 1 **characterized in that** in a cycle
the parameter levels of the reference materials used for performing the calibration upon which the quality control is based define a calibration range,
the parameter levels of all the reference materials used in said cycle define a total range, and
said calibration range is at least one fourth of said total range.

3. The method according to claim 2 **characterized in that** said calibration range is at least one third of said total range.

4. The method according to claim 1 **characterized in that** the method comprises three cycles A, B and C and includes three different reference materials, denoted reference material 1, reference material 2, and reference material 3 respectively,
wherein,
cycle A comprises performing a calibration of the sensor using reference material 2 and reference material 3 and performing a quality control of the sensor as calibrated using reference material 1,
cycle B comprises performing a calibration of the sensor using reference material 1 and reference material 2 and performing a quality control of the sensor as calibrated using reference material 3, and
cycle C comprises performing a calibration of the sensor using reference material 1 and reference material 3 and performing a quality control of the sensor as calibrated using reference material 2.

5. The method according to any of the preceding claims **characterized in that** the parameter is a blood parameter.

6. The method according to any of the preceding claims **characterized in that** the reference materials are fluids held in sealed containers.

7. The method according to any of the preceding claims **characterized in that** the reference materials represent more than one parameter.

8. An apparatus for determining a parameter in a test fluid comprising
a sensor sensitive to the parameter in the test fluid and providing a sensor response,
reference materials representing at least three different parameter levels of the parameter,
a programmable device for controlling the functioning of the apparatus
wherein
the programmable device controls the execution of the method of performing a calibration and quality control of the sensor according to any of the preceding claims.

9. The apparatus according to claim 8 **characterized in that** the apparatus is a blood analyzer.

## Patentansprüche

1. Verfahren zum Durchführen einer Kalibrierung und Qualitätskontrolle eines Sensors zum Bestimmen eines Parameters in einer Testflüssigkeit, wobei in dem Verfahren ein Kalibrier- und Qualitätskontrollzyklus wiederholt wird, wobei der Zyklus die folgenden Schritte umfasst:
Durchführen einer Kalibrierung des Sensors unter Verwendung von Referenzmaterialien, die unterschiedliche Parameterniveaus des Parameters darstellen,
Durchführen einer Qualitätskontrolle des kalibrierten Sensors unter Verwendung eines anderen Referenzmaterials, das ein anderes Parameterniveau des Parameters als in dem Kalibrierschritt darstellt,
wobei in dem Verfahren
das Referenzmaterial, das in dem Qualitätskontrollschritt eines Zyklus verwendet wurde, in dem Kalibrierschritt jedes der anderen Zyklen verwendet wird, wobei die Zyklen wiederholt werden, bis jedes der Referenzmaterialien einmal zur Qualitätskontrolle des Sensors verwendet wurde.

2. Verfahren nach Anspruch 1, **dadurch gekennzeichnet, dass** in einem Zyklus
die Parameterniveaus der Referenzmaterialien, die zum Durchführen der Kalibrierung verwendet werden, auf der die Qualitätskontrolle basiert, einen Kalibrierbereich definieren,
die Parameterniveaus aller Referenzmaterialien, die in dem Zyklus verwendet werden, einen Gesamtbereich definieren und
der Kalibrierbereich mindestens ein Viertel des Gesamtbereichs ist.

3. Verfahren nach Anspruch 2, **dadurch gekennzeichnet, dass** der Kalibrierbereich mindestens ein Drittel des Gesamtbereichs ist.

4. Verfahren nach Anspruch 1, **dadurch gekennzeichnet, dass** das Verfahren drei Zyklen A, B und C umfasst und drei unterschiedliche Referenzmaterialien beinhaltet, die als Referenzmaterial 1, Referenzmaterial 2 bzw. Referenzmaterial 3 bezeichnet werden,
wobei
Zyklus A das Durchführen einer Kalibrierung des Sensors unter Verwendung von Referenzmaterial 2 und Referenzmaterial 3 und das Durchführen einer Qualitätskontrolle des kalibrierten Sensors unter Verwendung von Referenzmaterial 1 umfasst,
Zyklus B das Durchführen einer Kalibrierung des Sensors unter Verwendung von Referenzmaterial 1 und Referenzmaterial 2 und das Durchführen einer Qualitätskontrolle des kalibrierten Sensors unter Verwendung von Referenzmaterial 3 umfasst und
Zyklus C das Durchführen einer Kalibrierung des Sensors unter Verwendung von Referenzmaterial 1 und Referenzmaterial 3 und das Durchführen einer Qualitätskontrolle des kalibrierten Sensors unter Verwendung von Referenzmaterial 2 umfasst.

5. Verfahren nach einem der vorhergehenden Ansprüche, **dadurch gekennzeichnet, dass** es sich bei dem Parameter um einen Blutparameter handelt.

6. Verfahren nach einem der vorhergehenden Ansprüche, **dadurch gekennzeichnet, dass** es sich bei den Referenzmaterialien um Flüssigkeiten handelt, die in verschlossenen Behältern aufbewahrt werden.

7. Verfahren nach einem der vorhergehenden Ansprüche, **dadurch gekennzeichnet, dass** die Referenzmaterialien mehr als einen Parameter darstellen.

8. Vorrichtung zum Bestimmen eines Parameters in einer Testflüssigkeit, wobei die Vorrichtung Folgendes umfasst:
einen Sensor, der für den Parameter in der Testflüssigkeit sensitiv ist und
eine Sensorreaktion liefert,
Referenzmaterialien, die mindestens drei unterschiedliche Parameterniveaus des Parameters darstellen,
ein programmierbares Gerät zum Steuern der Funktionsweise der Vorrichtung,
wobei
das programmierbare Gerät die Ausführung des Verfahrens zum Durchführen einer Kalibrierung und Qualitätskontrolle des Sensors nach einem der vorhergehenden Ansprüche steuert.

9. Vorrichtung nach Anspruch 8, **dadurch gekennzeichnet, dass** es sich bei der Vorrichtung um ein Blutanalysegerät handelt.

## Revendications

1. Procédé destiné à l'étalonnage et au contrôle de la qualité d'un capteur pour déterminer un paramètre dans un fluide d'essai, dans lequel procédé un cycle d'étalonnage et de contrôle de la qualité est répété, le cycle comprenant les étapes consistant à :
effectuer un étalonnage du capteur en utilisant des matériaux de référence représentant différents niveaux de paramètre du paramètre,
effectuer un contrôle de la qualité du capteur comme étalonné en utilisant un autre matériau de référence représentant un niveau de paramètre du paramètre autre qu'à l'étape d'étalonnage,
procédé dans lequel,
le matériau de référence utilisé à l'étape de contrôle de la qualité d'un cycle est utilisé à l'étape d'étalonnage de chacun des autres cycles, les cycles étant répétés jusqu'à ce que chacun des matériaux de référence ait été utilisé une fois pour le contrôle de la qualité du capteur.

2. Procédé selon la revendication 1, **caractérisé en ce que** dans un cycle
les niveaux de paramètre des matériaux de référence utilisés pour effectuer l'étalonnage sur lequel le contrôle de la qualité est basé définissent une plage d'étalonnage,
les niveaux de paramètre de tous les matériaux de référence utilisés dans ledit cycle définissent une plage totale, et
ladite plage d'étalonnage est au moins un quart de ladite plage totale.

3. Procédé selon la revendication 2, **caractérisé en ce que** ladite plage d'étalonnage est au moins un tiers de ladite plage totale.

4. Procédé selon la revendication 1, **caractérisé en ce que** le procédé comprend trois cycles A, B et C et comprend trois différents matériaux de référence, appelés matériau de référence 1, matériau de référence 2 et matériau de référence 3, respectivement,
dans lequel,
le cycle A consiste à effectuer un étalonnage du capteur en utilisant le matériau de référence 2 et le matériau de référence 3 et à effectuer un contrôle de la qualité du capteur comme étalonné en utilisant le matériau de référence 1,
le cycle B consiste à effectuer un étalonnage du capteur en utilisant le matériau de référence 1 et le matériau de référence 2 et à effectuer un contrôle de la qualité du capteur comme étalonné en utilisant le matériau de référence 3, et
le cycle C consiste à effectuer un étalonnage du capteur en utilisant le matériau de référence 1 et le matériau de référence 3 et à effectuer un contrôle de la qualité du capteur comme étalonné en utilisant le matériau de référence 2.

5. Procédé selon l'une quelconque des revendications précédentes, **caractérisé en ce que** le paramètre est un paramètre du sang.

6. Procédé selon l'une quelconque des revendications précédentes, **caractérisé en ce que** les matériaux de référence sont des fluides contenus dans des récipients étanches.

7. Procédé selon l'une quelconque des revendications précédentes, **caractérisé en ce que** les matériaux de référence représentent plus qu'un paramètre.

8. Appareil pour déterminer un paramètre dans un fluide d'essai comprenant
un capteur sensible au paramètre dans le fluide d'essai et fournissant une réponse du capteur,
des matériaux de référence représentant au moins trois différents niveaux de paramètre du paramètre,
un dispositif programmable pour commander le fonctionnement de l'appareil
dans lequel,
le dispositif programmable commande l'exécution du procédé destiné à l'étalonnage et au contrôle de la qualité du capteur selon l'une quelconque des revendications précédentes.

9. Appareil selon la revendication 8, **caractérisé en ce que** l'appareil est un analyseur de sang.
